# EUROPEAN PATENT APPLICATION

(11) **EP 2 476 418 A1**
(43) Date of publication of application: **18.07.2012**
(21) Application number: 12163980.1
(22) Date of filing: 21.12.2007
(51) Int. Cl.: A61K 31/4402, A61K 9/14, A61K 9/20, A61K 9/48, A61K 9/24, A61P 3/00, A61P 3/10

(54) **Pharmaceutical compound and composition for use in treating type II diabetes comprising rosiglitazone in a specific particle size**

(30) Priority: 21.12.2006 AU 2006907174
(62) Divisional of application: 07845435.2
(71) Applicant: Alphapharm Pty Ltd., Queensland 4300 (AU)
(72) Inventor: The designation of the inventor has not yet been filed
(74) Representative: Faivre Petit, Frédérique

(57) **Abstract**

A pharmaceutical composition comprising rosiglitazone or a pharmaceutically acceptable salt thereof wherein said rosiglitazone has a median particle size diameter of about 5 microns to about 20 microns.

## Description

### Technical Field

This invention relates to pharmaceutical compositions comprising Rosiglitazone, processes to prepare said compositions and uses of said compositions.

### Background Art

Rosiglitazone,5-[4-[2-(N-methyl-N-(2-pyridyl)amino)-ethoxy]benzyl]-2,4-thiazolidinedione maleate is a member of the thiazolidinedione class of antidiabetic agents and is a highly selective and potent agonist for the peroxisome proliferators-activated receptor-gamma (PPARy). In humans, PPAR receptors are found in key target tissues for insulin action such as adipose tissue, skeletal muscle, and liver. Rosiglitazone maleate is used for the management of type II diabetes mellitus, also called non-insulin-dependent diabetes mellitus (NIDDM). Rosiglitazone maleate is believed to act primarily by increasing insulin sensitivity and improving glycemic control while reducing circulating insulin levels.

Insulin resistance is a common feature characterizing the pathogenesis of type II diabetes.

Pharmaceutically active substances are commonly formulated into dosage forms to aid the delivery of small amounts thereof. The amount of pharmaceutically active substance that will be present in oral dosage forms can vary from a very small amount such as about 0.125mg up to larger amounts such as about 1000mg, depending on the pharmaceutically active substance being used and the pharmaceutical effective amount thereof. In order to be able to accurately administer these amounts of pharmaceutically active substance, the oral dosage form is often constituted of other pharmaceutically acceptable excipients that perform various functions depending on the dosage form and the mode of action required. These excipients have an effect on the method and rate of delivery of the pharmaceutically active substance to the patient.

Another aspect of pharmaceutical formulations that affects the rate of delivery or the bioavailability of the pharmaceutically active substance is the particle size thereof. This relationship between particle size and bioavailability is well known in the pharmaceutical industry and across a range of pharmaceutical products. In 1979, studies into the effect of crystal size on the bioavailability of Benoxaprofen were conducted (Biomed Mass Spectrom., 1979 Apr, 6(4), pp 173-8, Wolen RL et al; J. Pharm. Sci., 1979 Jul, 68(7), pp 850-2, Ridolfo AS et al). J. Pharm. Sci., 1980 Apr, 69(4), pp 391-4, Schoenwald RD & Stewart P disclose the effect of particle size on the ophthalmic bioavailability of dexamethasone stating that "A statistically significant rank-order correlation was observed between increasing drug levels and decreasing particle size." Other examples include American Journal of Veterinary Research, 1980 Dec, 41(12), pp 2095-2101, Shastri S et al; Clinical Pharmacokinetics, 1998 Feb, 34(2), pp 155-62, Miller DB & Spence JD; Current Med Res Opin, 2000, 16(2), pp 134-8, Guichard JP et al; J. Microencapsul., 2001 May-June, 18(3), pp 359-71, Demirel M et al; and Pharmaceutical Dev Technol, 2004, 9(1), pp 1-13, Rasenick N & Muller BW. Also refer to US 2002035119 A1 Rajiv, M et al; US 2003175338 A1 Manoj, KP et al; WO 03/082241 A3 Kumar, PM et al; WO 03/080056 A2 (Teva Pharmaceutical Industries Ltd); and US RE37516 E Grebow, PE et al that discuss the relationship between particle size and bioavailability of the pharmaceutically active substance.

WO 98/35681 (Novartis) further illustrates the effect of reducing the particle size of a drug with poor aqueous solubility. The formulations disclosed therein comprise micronised oxcarbazepine particles with a median particle size of between 2 - 12 microns (µm). Such particle size enhances the dissolution rate and consequently the bioavailability.

EP1448558A1 (SmithKline Beecham) discloses pharmaceutical compositions comprising 5- [4- [2- (N-methyl-N- (2-pyridyl) amino) ethoxy] benzyl] thiazolidine-2,4-dione hereinafter referred as rosiglitazone. The problem highlighted in this disclosure is that of bioavailability. As explained above it is well known in the art that an increase in the particle size of an active pharmaceutical ingredient (API) generally has a detrimental effect on the dissolution profile and consequently the bioavailability of the API to the body. Indeed EP1448558 solves this acknowledged bioavailability problem by providing compositions comprising rosiglitazone in particulate form wherein the median value of the mean volume diameter is between 500nm to 5 microns.

Further it is well known that the compositions comprising pioglitazone, a compound structurally very similar to rosiglitazone (figure 1), necessarily must comprise pioglitazone having a relatively small particle size. WO03080056 (Teva) discloses compositions comprising pioglitazone having a median particle size of 2µm to 7µm and as little as 10 volume percent of the particle less than 10µm. Again the problem to be overcome is that of providing formulations having increased bioavailability in line with the generally accepted view in the art that reducing the particle size of an API will ultimately improve the bioavailability of an API.

| Pioglitazone | Rosiglitazone |
|---|---|
| 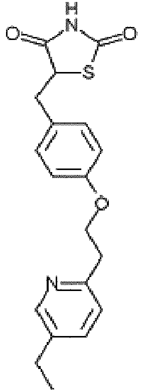 | 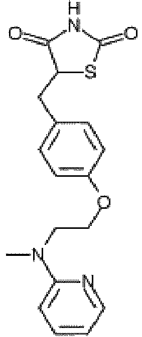 |

However there may be problems with the compositions comprising API having such small particle sizes. It has been noted that with compositions comprising such small particle sizes there are some instances in which particle size reduction fails to increase absorption rate and subsequently bioavailability. One reason might be that dissolution is not the rate limiting step. Additionally, micronisation sometimes increases the tendency of the particles to aggregate which may lead to a decrease in surface area. Further it has been reported that extremely small sizes may be inadvisable for some drug substances. Adsorbed air or crystal growth might act as dissolution rate limiting steps. Additionally, micronisation to such a small particle size requires greater energy input, more time and greater controls on the micronisation process to achieve the required range whilst reducing the amount of rejected material. Additionally, micronisation can lead to degradation of the API due the increase in reactive surface area resulting from micronisation.

Bioavailability can also be increased with the use of a surfactant or wetting agent. This helps to increase the solubility of the pharmaceutically active substance and thus bioavailability. However, there can be an undesired interaction between the pharmaceutically active substance and the wetting agent. Therefore, it is not always beneficial to use a wetting agent to increase the solubility and/or bioavailability of a pharmaceutically active substance.

WO2007/053904 relates to a general process for reducing particle size in order to allow for production of pharmaceutically active substances with a tightly controlled, reproducable distribution of median particle size, but is not specifically concerned with rosiglitzone and does not envisage the advantages any particulate median particle size for that active.

United States Patent Application Publication No. US2007/0148211 relates to a method for making an oral dosage form of a pharmaceutical agent which includes blending a pharmaceutical agent with particles of a preprocessed excipient to form a primary blend. The preprocessed excipient is formed by dissolving a bulking agent such as sugar and at least one non-friable excipient such as a waxy or liquid surfactant in a solvent and removing the solvent. Thereafter, the primary blend is milled to form a pharmaceutical formulation that includes micro-particles or nano-particles of the pharmaceutical agent. The admixture is characterised by particle size but not the pharmaceutical agent itself.

United States Patent Application Publication No US2006/0078622 relates to micro particles and/or nano particles containing a delivery agent and/or an active agent. There is a very broad disclosure of particle size ranges and simplification of insulin. There is specific discussion of rosiglitazone or anything specifically concerning the particle size of that active.

Thus there is always a need to provide improved formulations that overcome the problems of the prior art such as agglomeration and to provide effective or improved formulations that keep the beneficial properties of micronised particles, such as increase in aqueous solubility of the active ingredient, leading to an increase in bioavailability.

### Summary of the Invention

The inventors have surprisingly found that a pharmaceutical composition comprising rosiglitazone of a defined, larger median particle size overcomes the above problems with prior art. Further the claimed compositions are surprisingly effective and bioavailable in the face of the prior art teachings of EP1448558 and WO03080056 that collectively teach compositions comprising API with median particle sizes of less than about 7 µm. Further the prior art teaches that such particle sizes are necessary to provide bioavailable compositions.

Accordingly, there is provided a pharmaceutical composition according to the invention comprising rosiglitazone or a pharmaceutically acceptable salt thereof wherein said rosiglitazone has a median particle size diameter of about 5 microns to about 20 microns. In preferred embodiments the median particle size is about 7-15 microns, more preferably about 10 to about 12 microns and most preferred about 11 microns.

In another embodiment according to the invention, the composition is in the form of an oral dosage formulation. Preferably the oral dosage formulation is a tablet which in some embodiments is coated, in particular film-coated, but alternatively the oral dosage formulation is a capsule.

Certain embodiments of a formulation according to the invention comprise between about 1mg to 10mg rosiglitazone. Preferably 2mg to 8mg, but particularly preferred embodiments comprise 2mg, 4mg, 6mg or 8mg.

In a second aspect of a composition according to the invention, there is provided a tablet composition comprising between about 1 to 5% rosiglitazone or pharmaceutically acceptable salt thereof, in particular contained in a core, wherein said rosiglitazone has a median particle size diameter of about 5 microns to about 20 microns, preferably 7-15 microns, more preferably about 10 - 12 microns and most preferred about 11 microns and further comprising one or more pharmaceutically acceptable excipients. A particularly preferred embodiment of the second aspect provides a composition further comprising one or more of each of a diluent, a binder, a lubricant and a disintegrant. Preferably the diluent is one or more of lactose monohydrate and microcrystalline cellulose, the binder is hydroxypropyl methylcellulose, the disintegrant is sodium starch glycollate and the lubricant is magnesium stearate. Preferred amounts of each ingredient comprise between about 60-80% lactose monohydrate, 1-10% hydroxypropyl methylcellulose, 1-20% microcrystalline cellulose, 1-20% sodium starch glycollate and 1-10% magnesium stearate. Particularly preferred is a tablet composition comprising 2.7% rosiglitazone, 73.5% lactose monohydrate, 3% hydroxypropyl methylcellulose, 11% microcrystalline crystalline, 8% sodium starch glycollate and 1% magnesium stearate.

In alternative embodiments, the tablet composition is film-coated which coating in certain preferred embodiments comprises one or more coating polymers preferably the or each coating polymer is selected from film-coating systems such as Opadry^{®} or Opadry^{®} II by Colorcon, preferably Opadry^{®} II. In a particularly preferred embodiment, between about 1mg - 20mg of the coating system is present. In further preferred embodiments, there is provided a composition comprising between about 1mg to 10mg rosiglitazone, more preferably between 2mg to 8mg, most preferred is either 2mg or in alternative embodiments 4mg or 6mg or 8mg.

A third aspect of the present invention provides rosiglitazone or a pharmaceutically acceptable salt thereof having a median particle size diameter of about 5 microns to about 20 microns, preferably the median particle size diameter is about 7 microns to about 15 microns, most preferred is a median particle size diameter of about 10 microns to about 12 microns with particular preference of about 11 microns.

In a fourth aspect of the invention, there is provided a process for preparing a composition comprising rosiglitazone having a median particle size of between 5 - 20 microns, preferably 7 to 15 microns more preferably 10 - 15 microns or most preferred 11 microns, comprising admixing said rosiglitazone with one or more pharmaceutically acceptable carriers.

This process can comprise in particular:
i) admixing particulate rosiglitazone with one or more pharmaceutical excipients,
ii) forming a wet granulation mixture,
iii) granulating the mixture,
iv) drying the granules,
v) crushing the dried granules,
vi) blending the granules with one or more pharmaceutically acceptable excipients, and
vii) compressing granules into tablet form.

A fifth aspect comprises the use of rosiglitazone having a median particle size of between 5 - 20 microns, preferably 7 to 15 microns more preferably 10 - 15 microns or most preferred 11 microns to prepare a medicament for the treatment of type II diabetes.

A sixth aspect comprises the use of a pharmaceutical composition comprising rosiglitazone having a median particle size of between 5 - 20 microns, preferably 7 to 15 microns more preferably 10 - 15 microns or most preferred 11 microns to treat a human patient suffering from type II diabetes mellitus.

A seventh aspect provides a method of treating type II diabetes mellitus in a patient in need of such treatment comprising administering a pharmaceutical composition as described above.

For purposes of the present invention, the following terms are defined below.

"Pharmaceutically acceptable" refers to a substance that is useful in preparing a pharmaceutical composition that is generally non-toxic and is not biologically undesirable and includes those acceptable for veterinary use and/or human pharmaceutical use.

The term "composition" includes, but is not limited to, a powder, a solid dosage form, a suspension, an emulsion and/or mixtures thereof. The term composition is intended to encompass a product containing the specified ingredients in the specified amounts, as well as any product, which results, directly or indirectly, from a combination of the specified ingredients in the specified amounts. A "composition" may contain a single compound or a mixture of compounds. A "compound" is a chemical substance that includes molecules of the same chemical structure regardless of its three dimensional orientation. Thus, it may be used to indicate racemates, stereoisomers, or both.

The term "pharmaceutical composition" is intended to encompass a product including the active ingredient(s), pharmaceutically acceptable excipients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing the active ingredient, additional active ingredient(s), and pharmaceutically acceptable excipients.

The term "excipient" means a component of a pharmaceutical product that does not exhibit any therapeutic activity in or on a human or animal, such as filler, diluent, carrier, and so on. The excipients that are useful in preparing a pharmaceutical composition are preferably generally safe, non-toxic and neither biologically nor otherwise undesirable, and are acceptable for veterinary use as well as human pharmaceutical use. "A pharmaceutically acceptable excipient" as used in the specification and claims includes one or more of such excipients.

The particle size of the API that are the subject of the present invention maybe achieved using techniques common to those skilled in the art. Conventional comminution and de-agglomeration techniques may be used, for example grinding in an air-jet mill or impact mill, a ball mill, vibration mill, mortar mill or pin mill. Further techniques such as micro-fluidisation can also be used. Chemical techniques such as controlled precipitation/recrystallisation may also be employed.

Further, the known particle size analysis methods are suitable for determining the median particle size, for example particle size measurement using light, for example light-scattering methods or turbidimetric methods, sedimentation methods, for example pipette analysis using an Andreassen pipette, sedimentation scales, photo-sedimentometers or sedimentation in a centrifugal force field, pulse methods, for example using a Coulter counter, or sorting by means of gravitational or centrifugal force. Those methods are described, inter alia, in Voigt, loc. cit., pages 64-79.

### Detailed Description of the Invention

Tablets according to the invention may be manufactured by any means at the disposal of the skilled practitioner. Commonly used means include compressing rosiglitazone with conventional tabletting excipients to form a tablet core using conventional tabletting processes. Optionally the tablet cores may be coated. Coatings may comprise enteric release coatings and/or coatings that effect the release kinetics of rosiglitazone.

The tablet cores may be produced using conventional methods known in the art for example granulation methods, such as wet or dry granulation, with optional comminution of the granules and with subsequent compression and coating. Granulation methods are described, for example, in Voigt, loc. cit., pages 156-169.

Suitable excipients for the production of granules are, for example pulverulent fillers optionally having flow-conditioning properties, for example talcum, silicon dioxide, for example synthetic amorphous anhydrous silica acid of the Syloid^{®} X type (Grace), for example SYLOID^{®} 244 FP, microcrystalline cellulose, for example of the Avicel^{®} type (FMC Corp.), for example of the types AVICEL^{®} PH101, 102, 105, RC581 or RC 591, Emcocele^{®} type (Mendell Corp.) or Elcema type (Degussa); carbohydrates, such as sugars, sugar alcohols, starches or starch derivatives, for example lactose, dextrose, saccharose, glucose, sorbitol, mannitol, xylitol, potato starch, maize starch, rice starch, wheat starch or amylopectin, tricalcium phosphate, calcium hydrogen phosphate or magnesium trisilicate; particularly preferred is microcrystalline cellulose; binders, such as gelatin, tragacanth, agar, alginic acid, cellulose ethers, for example methylcellulose, carboxymethylcellulose or hydroxypropyl methylcellulose, polyethylene glycols or ethylene oxide homopolymers, especially having a degree of polymerisation of approximately from 2.0 x 10³ to 1.0 x 10⁵ and an approximate molecular weight of about from 1.0 x 10⁵ to 5.0 x 10⁶, for example excipients known by the name Polyoxe^{®} (Union Carbide), polyvinylpyrrolidone or povidones, especially having a mean molecular weight of approximately 1000 and a degree of polymerisation of approximately from 500 to 2500, and also agar or gelatine particularly preferred binder is hydroxypropyl methyllcellulose such as Hypromellose^{®}; surface-active substances, for example anionic surfactants of the alkyl sulphate type, for example sodium, potassium or magnesium n-dodecyl sulphate, n-tetradecyl sulphate, n-hexadecyl sulphate or n-octadecyl sulphate, of the alkyl ether sulphate type, for example sodium, potassium or magnesium n-dodecyloxyethyl sulphate, n-tetradecyloxyethyl sulphate, n-hexadecyloxyethyl sulphate or n-octadecyloxyethyl sulphate, or of the alkanesulfonate type, for example sodium, potassium or magnesium n-dodecanesulfonate, n-tetradecanesulfonate, n-hexadecanesulfonate or n-octadecane-sulfonate, or nonionic surfactants of the fatty acid polyhydroxy alcohol ester type, such as sorbitan monolaurate, monooleate, monostearate or monopalmitate, sorbitan tristearate or trioleate, polyoxyethylene adducts of fatty acid polyhydroxy alcohol esters, such as polyoxyethylene sorbitan monolaurate, monooleate, monostearate, monopalmitate, tristearate or trioleate, polyethylene glycol fatty acid esters, such as polyoxyethyl stearate, polyethylene glycol 400 stearate, polyethylene glycol 2000 stearate, especially ethylene oxide/propylene oxide block polymers of the Pluronicst (BWC) or Synperonice (ICI) type.

Granules may be produced in a manner known per se, for example using wet granulation methods known for the production of "built-up" granules or "broken-down" granules.

Methods for the formation of built-up granules may operate continuously and comprise, for example simultaneously spraying the granulation mass with granulation solution and drying, for example in a drum granulator, in pan granulators, on disc granulators, in a fluidised bed, by spray-drying or spray-solidifying, or operate discontinuously, for example in a fluidised bed, in a batch mixer or in a spray-drying drum.

Preferred are methods for the production of broken-down granules, which may be carried out discontinuously and in which the granulation mass first forms a wet aggregate with the granulation solution, which aggregate is then comminuted or formed into granules of the desired particle size and the granules then being dried. Suitable equipment for the granulation step are planetary mixers, low and high shear mixers, wet granulation equipment including extruders and spheronisers include, for example, apparatus from the companies Loedige, Glatt, Diosna, Fielder, Collette, Aeschbach, Alexanderwerk, Ytron,Wyss & Probst, Werner & Pfleiderer, HKD, Loser, Fuji, Nica, Caleva and Gabler.

The granulation mass consists of comminuted, preferably ground, rosiglitazone and the excipients mentioned above, for example pulverulent fillers, such as microcrystalline cellulose of the AVICEL^{®} type. AVICEL^{®} PH 102 is especially suitable. Depending on the method used, the granulation mass may be in the form of a premix or may be obtained by mixing the rosiglitazone into one or more excipients or mixing the excipients into the rosiglitazone. The wet granules are preferably dried, for example in the described manner by tray drying in an oven or drying in a fluidised bed dryer.

According to an alternative process variant, tablet cores are produced using the so-called compacting or dry granulation method in which the active ingredient is compressed with the excipients to form relatively large mouldings, for example slugs or ribbons, which are comminuted by grinding, and the ground material is compressed to form tablet cores.

Suitable excipients for the compacting method are preferably those which are suitable for the conventional direct compression methods, for example dry binders, such as starches, for example potato, wheat and maize starch, microcrystalline cellulose, for example commercial products available under the trademarks Avicel^{®}, Filtrak^{®}, Hewetene^{®} or Pharmace^{®}l, highly dispersed silicon dioxide, for example Aerosil^{®}, mannitol, lactose, and also polyethylene glycol, especially having a molecular weight of from 4000 to 6000, cross-linked polyvinylpyrrolidone (Polypiasdones XL or Kollidone^{®} CL), cross-linked carboxymethylcellulose (AcdisolX CMC-XL), carboxymethylcellulose [Nymcel, for example ZSB-10, (Nyma)], hydroxypropyl methylcellulose, for example the quality HPMC 603, carboxymethyl starch <RTI [ExplotabX (Mendell) or Primojele (Scholtens)], microcrystalline cellulose, for example Avicel^{®} PH 102, dicalcium phosphate, for example Emcompresse^{®} or talcum. The addition of small amounts of, for example, lubricants, such as magnesium stearate, is also advantageous.

Compression to form tablet cores may be carried out in conventional tabletting machines, for example EK-0 Korsch eccentric tabletting machines or rotary tabletting machines. The tablet cores may be of various shapes, for example round, oval, oblong, cylindrical etc., and various sizes, depending on the amount of rosiglitazone.

The following examples comprise rosiglitazone maleate having a median particle size of between 10 and 12 microns. Rosiglitazone maleate compound itself may be prepared according to known procedures such as those disclosed in U.S. Patent. Nos 5,002,953; 5,646,169; 5,741,803; and 6,288,095 of which the disclosures are incorporated herein by reference.

### Example 1

### Manufacture of Tablet

In one embodiment tablets according to the invention are prepared by:
i) admixing the particulate rosiglitazone (having a median particle size of about 11 microns) with one or more pharmaceutically acceptable excipients;
ii) forming a wet granulation mixture;
iii) granulating the mixture;
iv) drying the granules;
v) crushing the dried granules;
vi) blending the dried granules with one or more pharmaceutically acceptable excipients; and
vii) compressing the granules into tablet form.

Of course it will be appreciated by the skilled artisan that the tablets according to the invention may be coated by any means comprised in the art. A preferred method is detailed below.

### Coating of Tablets

1. Add the Opadry^{®} II Colour(s)to purified water and mix until the Opadry^{®} II colour(s) have dispersed.
2. Coat the tablets using the coating solution, aiming for an average tablet weight gain of approximately 3%.

Table 1 shows a tablet formulation according to the invention.

**TABLE 1**

| Ingredients | % |
|---|---|
| | |

| Active Ingredient | |
|---|---|
| Rosiglitazone maleate | 3.53 |
| | |

| Excipients | |
|---|---|
| Lactose monohydrate 300 | 73.47 |
| Hypromellose^{®} E3 | 3.00 |
| Microcrystalline Cellulose 101 | 11.00 |
| Sodium Starch Glycollate | 8.00 |
| Magnesium stearate | 1.00 |
| Purified Water | q.s. |
| Total | 100.00 |

Formulations prepared as per Table 1 exhibited improved and acceptable bioavailability and stability characteristics.

Of course it will be understood that the above examples are not intended to limit the scope of the invention. Various changes and modifications may be made by those skilled in the art without departing from the scope and spirit of the invention which is defined in the claims below.

## Claims

1. A pharmaceutical composition comprising rosiglitazone or a pharmaceutically acceptable salt thereof wherein said rosiglitazone has a median particle size diameter of about 5 microns to about 20 microns.

2. A pharmaceutical composition according to claim 1 wherein the rosiglitazone has a median particle size diameter of about 7 microns to about 15 microns.

3. A pharmaceutical composition according to claims to 1 or 2 wherein the rosiglitazone has a median particle size diameter of about 10 microns to about 12 microns.

4. A pharmaceutical composition according to claim 4 wherein the rosiglitazone has a median particle size diameter of about 11 microns.

5. A composition according to any preceding claim comprising between about 1mg to 10mg rosiglitazone, preferably 2mg to 8mg rosiglitazone.

6. A pharmaceutical composition according to any one of claims 1 to 5 comprising between about 1 to 5% rosiglitazone or pharmaceutically acceptable salt thereof in the form of a tablet.

7. A pharmaceutical composition according to claim 6 wherein the excipients comprise one or more of each of a diluent, a binder, a lubricant and a disintegrant.

8. A pharmaceutical composition according to claim 7 wherein the diluent is one or more of lactose monohydrate and microcrystalline cellulose, the binder is hydroxypropyl methylcellulose, the disintegrant is sodium starch glycollate and the lubricant is magnesium stearate.

9. A pharmaceutical composition according to claim 8 comprising between about 60-80% lactose monohydrate, 1-10% hydroxypropyl methylcellulose, 1-20% microcrystalline cellulose, 1-20% sodium starch glycollate, 1-10% magnesium stearate.

10. A pharmaceutical composition according to any one of claims 6 to 9 wherein the tablet is film-coated.

11. Rosiglitazone or a pharmaceutically acceptable salt thereof having a median particle size diameter of about 5 microns to about 20 microns.

12. Rosiglitazone according to claim 11 wherein the rosiglitazone has a median particle size diameter of about 7 microns to about 15 microns.

13. Rosiglitazone according to claim 12 wherein the rosiglitazone has a median particle size diameter of about 10 microns to about 12 microns.

14. Rosiglitazone according to claim 13 wherein the rosiglitazone has a median particle size diameter of about 11 microns.

15. Use of rosiglitazone according to any one of claims 11 to 14 to prepare a medicament for the treatment of type II diabetes mellitus.

16. Use of a pharmaceutical composition according to claims 1 to 10 to treat a human patient suffering from type II diabetes mellitus.
